(19)

Europäisches Patentamt
European Patent Office
Office européen des brevets

(11) **EP 1 787 624 A1**

(12) **EUROPEAN PATENT APPLICATION**
published in accordance with Art. 158(3) EPC

(43) Date of publication:
**23.05.2007 Bulletin 2007/21**

(51) Int Cl.:
***A61K 6/00*** (2006.01)

(21) Application number: **05780368.6**

(22) Date of filing: **17.08.2005**

(86) International application number:
**PCT/JP2005/015009**

(87) International publication number:
**WO 2006/019114 (23.02.2006 Gazette 2006/08)**

(84) Designated Contracting States:
**DE ES FR GB IT**

(30) Priority: **18.08.2004 JP 2004238702**

(71) Applicant: **Nichirei Foods Inc.**
**Tokyo 104-8402 (JP)**

(72) Inventors:
• **UCHIDA, Eriko,**
**c/o Research & Development Div.**
**Mihama-ku,**
**Chiba-shi,**
**Chiba 2618545 (JP)**
• **HANAMURA, Takayuki,**
**Research & Development Div.**
**Mihama-ku,**
**Chiba-shi,**
**Chiba 2618545 (JP)**

• **MAYAMA, Chisato,**
**c/o Research & Development Div.**
**Mihama-ku,**
**Chiba-shi,**
**Chiba 2618545 (JP)**
• **AOKI, Hitoshi,**
**c/o Research & Development Div.**
**Mihama-ku,**
**Chiba-shi,**
**Chiba 2618545 (JP)**

(74) Representative: **Woods, Geoffrey Corlett et al**
**J.A. KEMP & CO.**
**Gray's Inn**
**14 South Square**
**London WC1R 5JJ (GB)**

(54) **SKIN-LIGHTENING AGENT CONTAINING POLYPHENOL COMPOUND**

(57)    This invention provides a skin-whitening agent having satisfactory skin-whitening effects. This invention relates to: an inhibitor of melanin formation comprising, as an active ingredient, an acerola-derived polyphenol compound, an acerola polyphenol fraction, or another polyphenol compound; and an inhibitor of melanin formation further comprising, as an active ingredient, ascorbic acid or an ascorbic acid derivative. This invention also relates to a skin-whitening agent and a cosmetic, food or beverage, or pharmaceutical composition comprising the same.

EP 1 787 624 A1

**Description**

Technical Field

The present invention relates to an inhibitor of melanin formation, a skin-whitening agent, and a cosmetic, food or beverage, or pharmaceutical composition.

Background Art

Acerola is a tropical fruit of the genus *Malpighia* of the family *Malpighiaceae,* which is native to Caribbean Islands. Acerola fruit contains approximately 1,500 mg or more vitamin C per 100 g thereof, and it has become known as a plant that contains abundant vitamin C in recent years. Vitamin C is known to have various physiological and pharmacological effects, such as strengthening of tissue or capillary blood vessels, inhibition of melanin formation, and collagen formation, and vitamin C is extensively utilized in the cosmetic industry. Acerola fruit, which contains abundant naturally occurring vitamin C, has been utilized for cosmetics or other applications, in expectation of the tyrosinase inhibitory activity of vitamin C contained in acerola fruit extract (JP Patent No. 2,814,094). JP Patent Publication (Kokai) No. 10-316533 A (1998) teaches that vitamin C-free fermented acerola has whitening effects. Such effects are considered to be produced by organic acids or carboxylic acids, although the nature of the activity remains unknown.

In recent years, physiological activity of polyphenol components contained in acerola has also drawn attention. For example, the present inventors have discovered that acerola contains polyphenols including anthocyanin pigment and quercetin glycoside in amounts of about 100 mg to 300 mg per 100 g thereof. They have also discovered that such acerola polyphenols have effects of eliminating active oxygen (JP Patent Application No. 2003-375913), effects of inhibiting glucose absorption (JP Patent Application No. 2003-375323), and effects of maltase inhibition and activity of inhibiting AGE formation (JP Patent Application No. 2003-314207), and thus are effective in the prevention of lifestyle-related diseases, such as hyperglycemia or diabetic complications.

Human skin color varies depending on the melanin content of the epidermis, the blood flow in the capillary blood vessels, the horny layer thickness, and other conditions. In particular, the melanin content is reported as one of the most critical factors. In the process of melanin pigment generation, tyrosine is oxidized in the chromocytes (melanocytes) due to the action of tyrosinase, converted into dihydroxyphenylalanine (Dopa) and then dopaquinone, autooxidized to give dopachrome, and converted into 5,6-dihydroxyindole, followed by polymerization. Thus, melanin pigment is consequently generated. Pigment deposition, such as in the cases of blemishes and freckles, results from significantly enhanced melanin pigment generation caused by activated melanocytes, and such pigment deposition is a serious issue of concern for women and middle-aged and senior adults. Accordingly, cosmetic compositions for reducing the melanin content in the skin and for whitening the skin have been developed in recent years. Efforts for such development have been concentrated on the development of whitening agents for inhibiting the activity of tyrosinase, which plays a key role in melanin formation as described above. For example, inclusion of inhibitors of tyrosinase activity, such as vitamin C or vitamin C derivatives, arbutin, kojic acid, cysteine, and glutathione, in cosmetic products has been proposed. Skin-whitening agents utilizing placenta extracts, seaweed extracts (e.g., *Laminaria* and *Undaria),* and plant extracts (e.g., tea, aloe, and licorice) have been known.

Non-Patent Document 1 provides data demonstrating that neither delphinidin, cyanidin, and malvidin have effects of inhibiting tyrosinase activity.

Patent Document 1: JP Patent No. 2,814,094

Patent Document 2: JP Patent Publication (Kokai) No. 10-316533 A (1998)

Non-Patent Document 1: F. A. Badria and M. A. El Gayyar, Boll. Chim. Farmac. - Anno 140 - n. 4, pp. 267-271, 2001

Disclosure of the Invention

Object of the Invention

The aforementioned skin-whitening components or skin-whitening agents, however, cannot produce satisfactory skin-whitening effects. Stability or solubility thereof during production is insufficient, and safety thereof is still an issue of concern. Thus, such skin-whitening components or skin-whitening agents are not satisfactory for practical use.

Accordingly, an object of the present invention is to provide a skin-whitening agent having satisfactory skin-whitening effects.

Means for Attaining the Object

The present invention includes the following inventions.

(1) An inhibitor of melanin formation comprising, as an active ingredient, an acerola-derived polyphenol compound.
(2) An inhibitor of melanin formation comprising, as an active ingredient, an acerola polyphenol fraction obtained from acerola juice or acerola extract.
(3) An inhibitor of melanin formation comprising, as an active ingredient, an anthocyanidin compound, a glycoside thereof (excluding cyanidin-3-glucoside), or a physiologically acceptable salt thereof.
(4) The inhibitor of melanin formation according to (3), wherein the anthocyanidin compound is at least one member selected from the group consisting of cyanidin, pelargonidin, delphinidin, malvidin, and peonidin.
(5) The inhibitor of melanin formation according to (3) or (4), wherein the glycoside comprises an anthocyanidin compound and, bound thereto via a glycoside bond, a monosaccharide selected from the group consisting of rhamnose, glucose, galactose, mannose, xylose, ribose, arabinose, and glucuronic acid or an oligosaccharide comprising a plurality of the monosaccharides, which may be the same or different.
(6) The inhibitor of melanin formation according to any of (1) to (5), which further comprises, as an active ingredient, ascorbic acid or an ascorbic acid derivative.
(7) A skin-whitening agent comprising, as an active ingredient, the inhibitor of melanin formation according to any of (1) to (6).
(8) A cosmetic composition comprising the skin-whitening agent according to (7).
(9) A food or beverage composition comprising the skin-whitening agent according to (7).
(10) A pharmaceutical composition comprising the skin-whitening agent according to (7).
(11) A food or beverage composition comprising an acerola-derived polyphenol compound in an amount of 0.1 g to 30 g, and preferably 0.5 g to 10 g, per 100 g thereof.
(12) A food or beverage composition comprising an acerola-derived polyphenol compound and ascorbic acid or an ascorbic acid derivative, wherein the proportion of the weight of the acerola-derived polyphenol compound to the weight of the ascorbic acid or ascorbic acid derivative is 5:1 to 1:10, and preferably 3:1 to 1:2.

Effects of the Invention

The present invention provides a skin-whitening agent that suppresses skin blackening and is free of adverse effects. The skin-whitening agent of the present invention is useful in numerous fields of applications, such as the food, cosmetic, and pharmaceutical industries.
This description includes part or all of the contents as disclosed in the description and/or drawings of Japanese Patent Application No. 2004-238702, which is a priority document of the present application.

Brief Description of the Drawings

Fig. 1 shows the tyrosinase inhibitory activity of a C18-adsorbed fraction of acerola juice.
Fig. 2 shows the results of an experiment for confirming the activity of the C18-adsorbed fraction for inhibiting melanin formation with the use of B16 mouse melanoma cells.
Fig. 3 shows the tyrosinase inhibitory activity of cyanidin-3-rhamnoside (C3R).
Fig. 4 shows the tyrosinase inhibitory activity of pelargonidin-3-rhamnoside (P3R).

Preferred Embodiments of the Invention

Hereafter, the present invention is described in greater detail.
The present inventors have discovered that an acerola-derived polyphenol compound has effects of inhibiting melanin formation (and more specifically, effects of inhibiting tyrosinase activity). This has led to the completion of the present invention.
Specific examples of acerola-derived polyphenol compounds include anthocyanin pigments, such as cyanidin-3-rhamnoside and pelargonidin-3-rhamnoside, and quercetin glycosides, such as quercitrin (quercetin-3-rhamnoside), with anthocyanin pigments being preferable. Such polyphenols have excellent effects of inhibiting melanin formation in the form of mixtures of more than one polyphenol compound. When a single species of polyphenol compound is present in isolation, and particularly when pelargonidin-3-rhamnoside is present in isolation, more potent effects of inhibiting melanin formation are exhibited. Thus, such state is preferable.

Parts of acerola that serve as origins of polyphenol compounds are not particularly limited. For example, acerola fruit, root, stem, and leaf can be used. Extraction of polyphenol compounds from acerola fruit is particularly preferable. The term "fruit" used herein refers to all portions of fruit, including edible parts and seeds.

Polyphenol compounds can be extracted from acerola by conventional techniques. For example, polyphenol compounds can be obtained by adequately purifying acerola juice squeezed from acerola fruit or extracts from the aforementioned sites.

Examples of the methods for obtaining acerola extracts include an extraction method involving the use of water or an organic solvent, an extraction method involving the use of water containing a hydrophilic organic solvent such as alcohol, a method wherein an organic solvent is used to obtain a crude extract and a synthetic adsorbent is then allowed to react with the crude extract to obtain an extract containing flavonoids via adsorption, and an extraction method involving the use of a supercritical fluid. The type of water that can be used as an extraction solvent is not particularly limited, and it may be pure water or purified water, for example. When an organic solvent is used for extraction, a hydrophilic or hydrophobic organic solvent may be used. Examples of hydrophilic organic solvents include conventional organic solvents, for example, alcohols, such as methyl alcohol, ethyl alcohol, glycerin, propylene glycol, and 1,3-butylene glycol, acetone, tetrahydrofuran, acetonitrile, 1,4-dioxane, pyridine, dimethyl sulfoxide, N,N-dimethylformamide, and acetic acid. Such hydrophilic organic solvents may be used in combination with water. Examples of hydrophobic organic solvents include conventional organic solvents, such as hexane, cyclohexane, carbon tetrachloride, chloroform, dichloromethane, 1,2-dichloroethane, diethyl ether, ethyl acetate, benzene, and toluene. These hydrophilic and hydrophobic organic solvents can be used solely or in combinations of two or more.

Conditions for extraction are not particularly limited. The temperature range is preferably between 5°C and 90°C, and more preferably between 20°C and 40°C. When extraction is carried out at a high temperature, hydrolysis may occur, particularly with the extraction of polyphenols that form glycosides. Thus, polyphenol constituents extracted at a low temperature may differ from those extracted at a high temperature, although such difference would not affect at all functionality or activity intensity of polyphenol. The duration of the extraction process is preferably about 1 to 10 hours, and more preferably about 1 to 2 hours, and the amount of solvent used for extraction is preferably 1 to 20 times greater than that of the starting material by mass.

After extraction, the extraction residue is removed by filtration or centrifugation to obtain an extract. The resulting extract can be concentrated according to need. The extraction residue may be further subjected to the same extraction procedure.

The resulting extract often contains abundant saccharides or organic acids. Accordingly, it is preferable to carry out a step of purification in order to remove such substances. Purification may be carried out via, for example, normal-phase or reverse-phase chromatography, ion-exchange chromatography, or gel filtration. These techniques may be carried out in combination.

Polyphenol compounds can be isolated and purified from acerola juice or extract via any method without particular limitation. Examples thereof include HPLC, chromatography using a synthetic absorbent, ion-exchange chromatography, and gel filtration. Chromatography using a synthetic absorbent is particularly preferable. In such case, the extract is preferably eluted with a 10% to 50% ethanol solution, for example. Since an anthocyanin pigment is stabilized under acidic conditions, it is particularly preferable that hydrochloric acid or acetic acid be added to the eluate for acidification.

The acerola polyphenol fraction obtained from acerola juice or extract also has effects of inhibiting melanin formation. The term "acerola polyphenol fraction" used herein refers to a fraction containing a polyphenol compound eluted from the acerola juice or extract by any of the various aforementioned chromatography techniques under the aforementioned conditions. Acerola polyphenol fraction includes an eluate, a concentration thereof, and a dehydration product thereof.

The present inventors further discovered that, in addition to acerola-derived compounds, various anthocyanidin compounds, glycosides thereof (excluding cyanidin-3-glucoside), or physiologically acceptable salts thereof have effects of inhibiting melanin formation (more particularly, effects of inhibiting tyrosinase activity). The present invention also relates to an inhibitor of melanin formation comprising, as an active ingredient, any of such components.

Specific examples of the aforementioned anthocyanidin compounds include cyanidin, pelargonidin, delphinidin, malvidin, peonidin, and petunidin. Cyanidin, pelargonidin, delphinidin, malvidin, and peonidin are particularly preferable.

In the present invention, the term "anthocyanidin compound" also refers to a derivative of an anthocyanidin compound having substantially the same effects of inhibiting melanin formation. Examples of such derivative include acylated and deoxidized forms of anthocyanidin compounds.

The glycoside preferably comprises an anthocyanidin compound and, bound thereto via a glycoside bond (an O-glycoside bond, in general), a monosaccharide selected from the group consisting of rhamnose, glucose, galactose, mannose, xylose, ribose, arabinose, and glucuronic acid (and more preferably rhamnose) or an oligosaccharide (e.g., disaccharide or trisaccharide) comprising a plurality of the monosaccharides, which may be the same or different. A glycoside bond is generally formed by a bond between a hydroxyl group at position 3, 3', 5, or 7 of the anthocyanidin compound with a saccharide.

The glycoside is not limited to a conjugate of an anthocyanidin molecule and a saccharide molecule. The same or a different plurality of saccharide molecules may be bound to an anthocyanidin molecule.

Among the aforementioned anthocyanidin compounds and glycosides thereof, cyanidin-3-rhamnoside, pelargonidin-3-rhamnoside, cyanidin, pelargonidin, delphinidin, peonidin, and malvidin are particularly preferable. The present invention has been made based on findings completely opposite from those described in Non-Patent Document 1 such that neither delphinidin, cyanidin, or malvidin has effects of inhibiting tyrosinase activity. Thus, a person skilled in the art would be unable to readily conceive of the present invention.

Examples of the physiologically acceptable salts include acid addition salts with organic or inorganic acids, such as hydrochloride, hydrobromate, sulfate, bisulfate, phosphate, acidic phosphate, acetate, oxalate, maleate, fumarate, succinate, lactate, tartrate, benzoate, citrate, gluconate, saccharate, cyclohexyl sulfamate, methane sulfonate, p-toluenesulfonate, and naphthalenesulfonate.

The content of the acerola-derived polyphenol compound in the inhibitor of melanin formation according to the present invention is not particularly limited, as long as effects of inhibiting melanin formation can be effectively produced. For example, such content may be 0.1 g to 30 g, and preferably 0.5 g to 10 g, per 100 g of the agent.

More surprisingly, the present inventors have discovered that superior effects of inhibiting melanin formation can be produced when the acerola-derived polyphenol compound, acerola polyphenol fraction, or any of the aforementioned polyphenol compounds is used in combination with ascorbic acid. This has led to the completion of the present invention. It has been heretofore known that ascorbic acid (vitamin C) has effects of inhibiting melanin formation caused by tyrosinase, mainly because of its effects of reduction. In the present invention, effects of the acerola-derived polyphenol compound and ascorbic acid for inhibiting melanin formation were discovered to be synergistically improved upon combination thereof. It was also confirmed that a combination of the acerola-derived polyphenol compound and ascorbic acid can produce the same effects with smaller amounts than are necessary with the independent use of such substances. A derivative of ascorbic acid may also be used, and examples thereof include: metal salts of ascorbic acid, such as potassium, sodium, magnesium, or calcium salt; ascorbic acid phosphate ester salts, such as ascorbic acid phosphate ester potassium salt, ascorbic acid phosphate ester magnesium salt, and ascorbic acid phosphate ester calcium salt; and ascorbic acid sulfate ester salts, such as ascorbic acid sulfate ester sodium salt and ascorbic acid sulfate ester potassium salt. When the inhibitor of melanin formation according to the present invention contains an acerola-derived polyphenol compound and ascorbic acid or an ascorbic acid derivative, the proportion of the weight of the former to the weight of the latter is preferably 4:1 to 1:8, although the proportion is not limited to such range.

The inhibitor of melanin formation according to the present invention has the skin-whitening effects as described in the examples. Specifically, the present invention also relates to a skin-whitening agent comprising, as an active ingredient, the aforementioned inhibitor of melanin formation.

Typically, the skin-whitening agent according to the present invention is expected to be effective in prevention of skin aging or in prevention or treatment of skin cancer, as well as exhibiting effects of skin whitening as an ingredient of a cosmetic, food or beverage, or pharmaceutical composition.

A cosmetic composition can be used in a general form, such as a cosmetic cream, emulsion, lotion, essence, facial mask, powder, lip balm, lipstick, make-up base, foundation, sun protector, bath agent, body-wash, body lotion, soap, cleansing foam, ointment, gel, or aerosol. In addition to the skin-whitening agent of the present invention, a cosmetic composition can adequately comprise oil, surfactant, alcohol, moistening agent, antioxidant, skin-whitening agent, ultraviolet absorber, antibacterial and antifungal agents, pigment, dye, and perfume, within a range such that the desired effects of the present invention are not deteriorated. The content of the acerola-derived polyphenol compound in the cosmetic composition of the present invention is not particularly limited, as long as the use thereof as a cosmetic product can effectively produce effects of inhibiting melanin formation. For example, it may be 0.001 % to 4% by weight, and preferably 0.04% to 0.8% by weight. When the cosmetic composition of the present invention contains an acerola-derived polyphenol compound in combination with ascorbic acid or an ascorbic acid derivative, the proportion of the weight of the former to the weight of the latter is preferably 5:1 to 1:10, and more preferably 3:1 to 1:2, although the proportion is not limited to such range.

Examples of forms of food or beverage compositions include beverage, solid food, and semisolid food products, and such compositions may also be in the form of foods for specified health uses. Specific examples of beverages include fruit juice beverages, soft drink beverages, and alcoholic beverages. Alternatively, a beverage may be in the form of a powder that is diluted with water before ingestion. Solid food products can be of various forms, and examples thereof include tablets including candies and troches, sugar-coated tablets, granules, powdered beverages, powdery food products such as powdered soup, block-shaped confectioneries such as biscuits, capsules, and gels. Forms of semisolid food products include, for example, pastes such as jams and gum such as chewing gum. In addition to the skin-whitening agent of the present invention, these food or beverage compositions can comprise various ingredients that are usually used as starting materials for food, within a range such that the desired effects of the present invention are not deteriorated. Examples of such ingredients include water, alcohols, sweeteners, acidulants, colorants, preservatives, perfumes, and excipients. These ingredients can be used solely or in combinations of two or more. The content of the acerola-derived polyphenol compound in a food or beverage composition of the present invention is not particularly limited, as long as the ingestion thereof can effectively produce effects of inhibiting melanin formation. For example, it may be 0.1 g to 30 g, and preferably 0.5 g to 10 g, per 100 g of the food or beverage composition. When the food or beverage composition of the present invention contains an acerola-derived polyphenol compound in combination with ascorbic acid or an ascorbic acid derivative, the proportion of the weight of the former to the weight of the latter is preferably 5:1 to 1:10, and more preferably 3:1 to 1:2, although the proportion is not limited to such range.

The form of a pharmaceutical composition is not particularly limited, and it may be adequately selected according to need. A dosage form can be a preparation for oral or parenteral administration. Examples of dosage forms for oral preparations include powders, tablets, granules, fine granules, liquids, capsules, pills, troches, liquid mixtures for internal use, suspensions, emulsions, syrups, and elixirs. Examples of parenteral preparations include transnasal, enteral, and transdermal preparations, and parenteral preparations can be in the form of, for example, injections, drops, suppositories, inhalants, transdermal absorbents, transmucosal absorbents, adhesive preparations, or ointments. These preparations can be used alone or in combinations of two or more in accordance with symptoms. These preparations can be prepared by conventional techniques. In such a case, carriers, excipients, binders, preservatives, oxidative stabilizers, disintegrators, lubricants, taste corrigents, or diluents can be adequately selected from among conventional substances. The content of an acerola-derived polyphenol compound in the pharmaceutical composition of the present invention is not particularly limited, as long as the administration thereof can effectively produce effects of inhibiting melanin formation. For example, it may be 0.1 g to 30 g, and preferably 0.5 g to 10 g, per 100 g of the pharmaceutical composition. When the pharmaceutical composition of the present invention contains an acerola-derived polyphenol compound in combination with ascorbic acid or an ascorbic acid derivative, the proportion of the weight of the former to the weight of the latter is preferably 5:1 to 1:10, and more preferably 3:1 to 1:2, although the proportion is not limited to such range.

Hereafter, the present invention is described in greater detail with reference to the following examples, although the technical scope of the present invention is not limited to these examples.

Examples

[Example 1]

Preparation of C18-adsorbed fraction

Seeds were separated from 1 kg of acerola fruits, purified water was added to the remnant in an equivalent amount, and the resulting mixture was homogenized. The resulting suspension was centrifuged, filtered, applied to C18 cartridge columns (Sep-Pak Vac 35cc C18 cartridge columns, Waters), washed with distilled water, and eluted with a 0.2% TFA/methanol solution. The eluate was lyophilized to give 1.2 g of powder. This powder was designated as an acerola polyphenol fraction (C 18-adsorbed fraction). The components of the C 18-adsorbed fraction were analyzed, and as a result, this fraction was found to contain no glucose, fructose, or vitamin C. Subsequently, polyphenol content was assayed by the Folin-Denis method. An analytical curve was derived using catechin as a standard reference material. As a result, the total polyphenol content of the resulting C18-adsorbed fraction was found to be 40.7%. At first, the total polyphenol content of this fraction was calculated as 22%; however, this value was an incorrect value resulting from an error at the time of assay or during the derivation of the analytical curve. Therefore, the same sample was again subjected to accurate assay and an analytical curve was again derived. As a result, an adequate value was found to be 40.7%, as described above.

[Example 2]

Preparation of acerola-derived polyphenol

Seeds were separated from acerola fruits, the remaining edible parts were homogenized, and 3x amount of methanol was added thereto, followed by 1-hour extraction at 27°C. This procedure was carried out twice, and the resulting extract was centrifuged, filtered, lyophilized, and then diluted in distilled water again. The resulting solution was applied to the C18 cartridge columns (Sep-Pak Vac 35cc C18 cartridge columns, Waters) and thoroughly washed in 10% methanol. Thereafter, a fraction eluted with a 0.1 % TFA-containing 20% methanol solution and a fraction eluted with a 0.1% TFA-containing 30% methanol solution were obtained. The fractions were further purified via HPLC using reversed-phase columns, and cyanidin-3-rhamnoside (hereafter abbreviated as "C3R") and pelargonidin-3-rhamnoside (hereafter abbreviated as "P3R") were obtained from the fraction after it was eluted with 20% methanol.

6

[Example 3]

Inhibition of tyrosinase activity by C18-adsorbed fraction

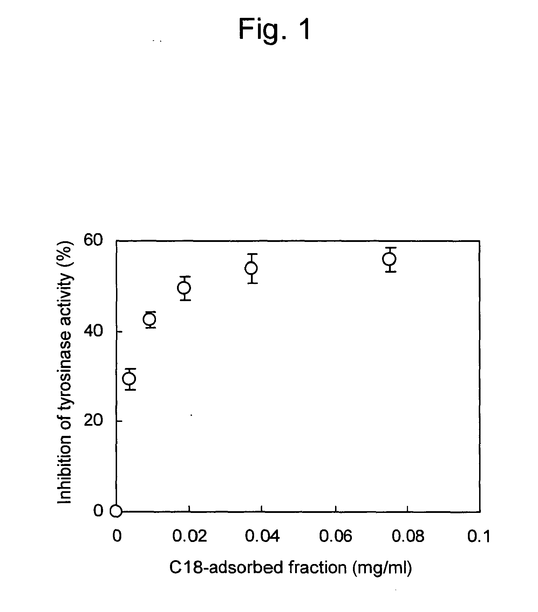

1/15M phosphate buffer (70 μl, pH 6.8), 2 μl each of sample solutions of the C18-adsorbed fraction obtained in Example 1 dissolved in dimethyl sulfoxide (DMSO) at various concentrations, and 70 μl (10 U) of mushroom tyrosinase (Calzyme Laboratories) dissolved in the buffer were mixed, and the mixture was incubated at 25°C for 10 minutes. Thereafter, 70 μl of 10 mM L-Dopa was added to initiate the reaction, the absorbance at 475 nm was assayed every 20 seconds for 2 minutes, and the inhibition (%) of tyrosinase activity was calculated using the following equation. The results are shown in Fig. 1.
Inhibition of tyrosinase activity (%) = (1 - a/b) x 100

a: changes in the absorbance per unit of time of reaction solution to which the sample solution was added
b: changes in the absorbance per unit of time of reaction solution to which DMSO instead of sample solution was added

As is apparent from Fig. 1, effects of inhibiting tyrosinase activity were observed in the C18-adsorbed fraction.
Subsequently, polyvinylpyrrolidone (PVPP) resin capable of adsorbing polyphenol was added to an aqueous solution of 0.132% of the C18-adsorbed fraction obtained in Example 1 to a concentration of 5%, and the solution was agitated for 1.5 hours, followed by centrifugation (at 1,800 g for 15 minutes) to obtain a supernatant. Further, PVPP resin was added to the supernatant to a concentration of 1%, and the resultant was also agitated for 1.5 hours, followed by centrifugation to obtain a supernatant. This supernatant was lyophilized, the product was designated as a non-PVPP-adsorbed fraction, and the inhibition of tyrosinase activity thereof was also inspected in the same manner as described above at 37°C. The sample was dissolved in 1/15M phosphate buffer (pH 6.8) instead of DMSO. The polyphenol content was assayed in the same manner as in Example 1. The results are shown in Table 1. As is apparent from Table 1, the presence of polyphenol components is considered to be strongly correlated with the degree of inhibition of tyrosinase activity of the C 18-adsorbed fraction.

Table 1

|  | Polyphenol content (%) | Inhibition of tyrosinase activity (%) |
| --- | --- | --- |
| C18-adsorbed fraction | 40.7 | 87.8 |
| Non-PVPP-adsorbed fraction | 3.8 | 27.1 |

[Example 4]

Experiment for inspecting effects of C18-adsorbed fraction for inhibiting melanin formation using B16 mouse melanoma cells

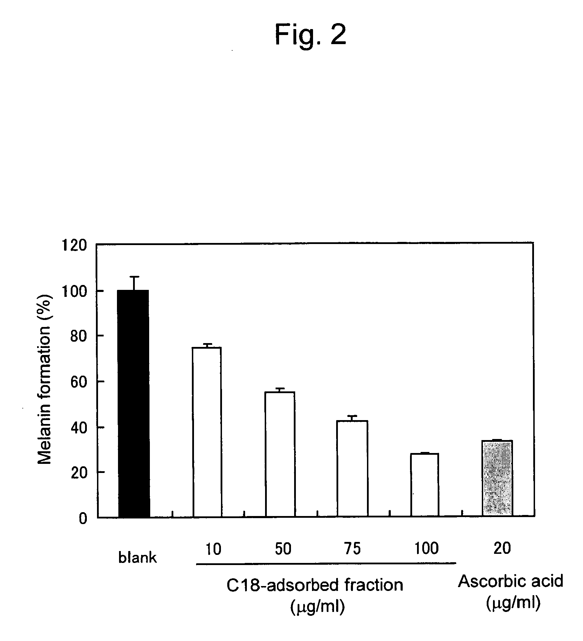

Skin-whitening effects of the C18-adsorbed fraction prepared in Example 1 were examined at the cellular level using B16 mouse melanoma cells. According to this method, with the use of animal cells, effects of inhibiting the generation of melanin pigment and the influence on cell growth can be examined under an environment more similar to the *in vivo* environment, compared with other methods.
B16 mouse melanoma cells were sowed in a petri dish at a cell density of $1 \times 10^5$ cells/ml, and the cells were cultured at 37°C in the presence of 5% $CO_2$ for 2 days. The culture solution was removed, test media each comprising 10, 50, 75, or 100 μg/ml of the C18-adsorbed fractions were then added in amounts of 10 ml per dish, and culture was carried out at 37°C in the presence of 5% $CO_2$ for an additional 3 days. After the culture solution was removed, cells were treated with trypsin solution and harvested from the dish. They were then centrifuged, suspended in PBS, and centrifuged again. A 1N sodium hydroxide solution was added to the cell pellet obtained by removing the supernatant, the mixture was heated to dissolve the melanin pigment, and cell-derived fibrous substances were removed by filtration. The dissolved melanin pigment was assayed using an absorption spectrometer, and the protein content was assayed using the DC-Protein Assay Kit (Bio-Rad).
For a blank group, the same test was performed using 10% FBS/DME medium instead of the medium containing C18-adsorbed fractions. As a positive control group, the same test was performed using a medium containing 20 μg/ml of ascrobic acid instead of C18-adsorbed fractions.
The biosynthesized melanin content per mg of protein of the blank group was designated as 100%, and the melanin formation of each group comprising C18-adsorbed fractions was calculated as a percentage thereof by the following equation. The results are shown in Fig. 2.

Melanin formation (%) = ([average melanin content per mg of total protein in group comprising C18-adsorbed fraction]/[average melanin content per mg of total protein of blank group]) x 100

According to the results shown in Fig. 2, the C18-adsorbed fraction was found to have effects of inhibiting melanin formation equivalent to those of ascorbic acid. Since no change was observed in the total protein content in the concentration range employed in this test, the addition of the C18-adsorbed fraction was considered to impose no influence on cell growth.

[Example 5]

Inhibition of tyrosinase activity by acerola-derived polyphenol component

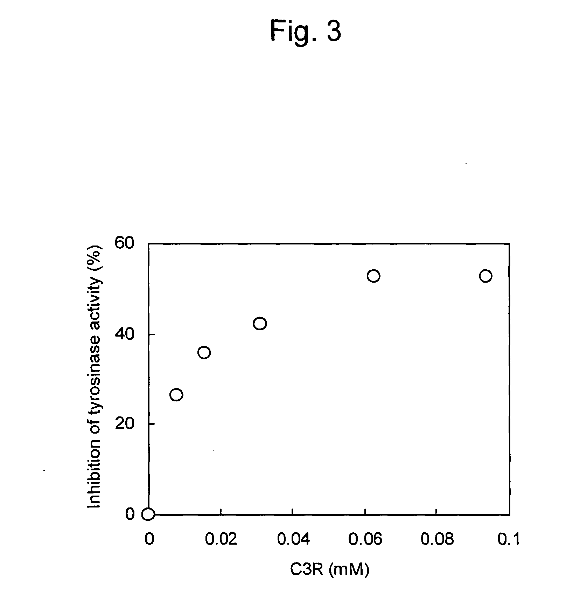

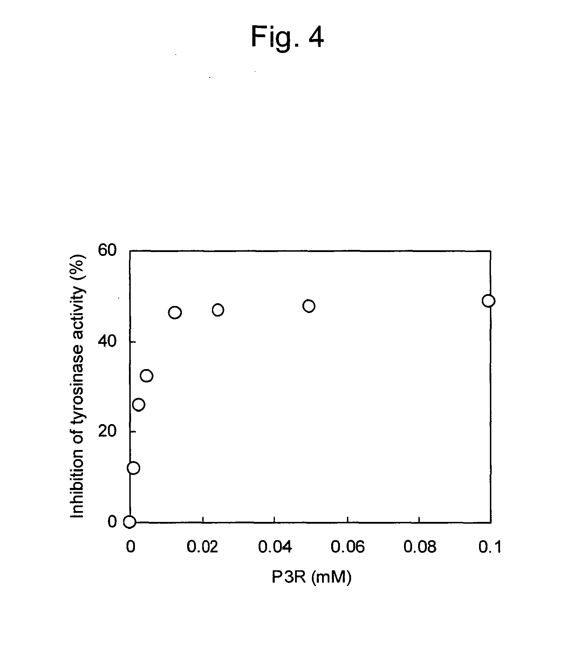

Two kinds of acerola-derived polyphenols, C3R and P3R obtained in Example 2, were subjected to the test for examining inhibition of tyrosinase activity in accordance with the procedure of Example 3. The results are shown in Fig. 3 and in Fig. 4. As shown in Fig. 3 and in Fig. 4, C3R and P3R were found to have effects of inhibiting tyrosinase activity. The concentrations of C3R, P3R, and kojic acid at which 50% of tyrosinase activity was inhibited are shown in Table 2. Kojic acid, which is well known as a tyrosinase inhibitor, was used as a positive control. Since kojic acid inhibits tyrosinase activity and suppresses melanin formation, kojic acid was added to cosmetic products in the past. As a result of animal tests, however, kojic acid was found to be a potential cause of liver cancer. At present, accordingly, use thereof for cosmetics is banned in accordance with a notice issued by the Ministry of Health, Labour and Welfare in March, 2001. As shown in Table 2, effects of C3R for inhibiting tyrosinase activity were equivalent to those of kojic acid, and such effects of P3R were about 6 times greater than those of kojic acid. These results indicate that C3R and P3R can be utilized as skin-whitening agents prepared from natural products.

Table 2

| | Concentration at which 50% of tyrosinase activity is inhibited ($\mu$M) |
| --- | --- |
| C3R | 33 |
| P3R | 5.7 |
| Kojic acid | 38 |

[Example 6]

Synergistic effects with ascorbic acid

It has been heretofore known that ascorbic acid (vitamin C) has effects of inhibiting melanin formation caused by tyrosinase, mainly as a result of its effects of reduction. The synergistic effects of inhibiting tyrosinase activity attained by ascorbic acid in combination with the C18-adsorbed fraction were examined. Inhibition of melanin formation resulting from the reduction effects of ascorbic acid can also be evaluated using "inhibition of tyrosinase activity (%)" as shown in Example 3. The results of the evaluation are shown in Table 3. As is apparent from Table 3, the addition of a minor amount of ascorbic acid can produce synergistic effects of the C18-adsorbed fraction in combination with ascorbic acid, and a minor amount of C18-adsorbed fraction can produce satisfactory effects of inhibiting melanin formation.

Table 3

| | Inhibition of tyrosinase activity (%) |
| --- | --- |
| Ascorbic acid (33.3 $\mu$M) | 5.49 |
| Ascorbic acid (16.7 $\mu$M) + C18-adsorbed fraction (0.15 mg/ml) | 57.14 |
| C18-adsorbed fraction (0.3 mg/ml) | 64.94 |

[Example 7]

Mass-production of acerola polyphenol fractions 1

Seeds were separated from acerola fruits, and the remaining acerola fruits (80 kg) were squeezed to obtain juice and residues. The residues were washed with distilled water, the wash was mixed with juice, and the mixture was then lyophilized. The obtained sample was dissolved in distilled water again, the solution was applied to a synthetic adsorption resin (Amberlite XAD, 16 HP columns), and the resultant was washed with distilled water, followed by elution with a 0.2% TFA/methanol solution. Thereafter, the eluate was lyophilized to obtain 96 g of an acerola polyphenol fraction. The components of this fraction were analyzed. As a result, this fraction was found to contain no glucose, fructose, or vitamin C, and its total polyphenol content was found to be about 40% (by the Folin-Denis method).

[Example 8]

Mass-production of acerola polyphenol fractions 2

Seeds were separated from acerola fruits, and the remaining acerola fruits were squeezed to obtain juice and residues. The obtained juice (8 t) was mixed with an equivalent amount of distilled water, the mixture was thoroughly agitated, the resulting solution was applied to a synthetic adsorption resin (Amberlite XAD, 7HP columns), and the resultant was washed with distilled water, followed by elution with a 70% ethanol solution containing malic acid. Thereafter, the eluate was lyophilized to obtain 5.9 kg of powder of an acerola polyphenol fraction. The total polyphenol content of this fraction was found to be about 40% (by the Folin-Denis method). The obtained powder of the acerola polyphenol fraction contained 1% of the malic acid that had been added to the eluate.

[Example 9]

Beverage comprising acerola polyphenol

Based on the results of Examples 3 to 6, beverages comprising acerola polyphenols are expected to prevent or improve skin dullness or blemishes. A method for preparing such beverages is not particularly limited. For example, 200 ml of a beverage can be prepared with the use of 250 mg of the acerola polyphenol fraction prepared in Example 8 by a conventional technique.

The amount and duration of beverage ingestion necessary for attaining effects of making skin dullness or blemishes less noticeable can be adequately determined. For example, it is preferable that 200 ml of the above beverage be ingested per day over a period of 12 weeks.

[Example 10]

Skin-whitening effects of acerola polyphenol-containing lotion on humans

Acerola polyphenol-containing lotions having the formulations shown in Table 4 were prepared. Lotions were prepared in a hot water bath. The composition of the test product A was identical to that of a control product. The test product A was given to each subject as a placebo sample in the following test 1.

Table 4

| Formulations of test products for evaluation (unit = % by weight) | | | | | |
|---|---|---|---|---|---|
| Ingredients | Control | Test Products | | | |
| | | A | B | C | D |
| C18-adsorbed fraction (Example 8) | 0 | 0 | 0.01 | 0.1 | 0.5 |
| Ethanol | 9 | 9 | 9 | 9 | 9 |
| Glycerin | 6 | 6 | 6 | 6 | 6 |
| 1,3-Butylene glycol | 5.5 | 5.5 | 5.5 | 5.5 | 5.5 |
| Polyoxyethylene sorbitan monolaurate | 1.5 | 1.5 | 1.5 | 1.5 | 1.5 |
| Polyoxyethylene lauryl ether | 0.5 | 0.5 | 0.5 | 0.5 | 0.5 |
| Mannitol | 0.5 | 0.5 | 0.5 | 0.5 | 0.5 |
| Purified water | Balance | Balance | Balance | Balance | Balance |

Test 1: Sensations during use

A control product and test products A to D (5 types in total) were applied to the backs of the hands of 10 subjects, and the subjects were subjected to an inquiry survey regarding the sensations they experienced during use.

In the inquiry survey, 5 items were the subjects of inquiry, i.e., color, fragrance, smoothness, moistness, and clarity, and the score of the control product was designated as point 0. The subjects were requested to grade each test product from -3 to 3 by points, with higher scores for more preferable products, and a means was determined to evaluate the products. The results are shown in Table 5.

Table 5

| | Test products | | | |
| Evaluation items | A | B | C | D |
|---|---|---|---|---|
| Color | 0.6 | 0.8 | 0.7 | -0.2 |
| Fragrance | 0.2 | 0.7 | 1.1 | 1.3 |
| Smoothness | 0.0 | 0.0 | 0.4 | 0.8 |
| Moistness | 0.6 | 0.6 | 1.3 | 1.7 |
| Clarity | 0.1 | 0.4 | 1.1 | 1.2 |

As is apparent from Table 5, test products containing C18-adsorbed fractions, and in particular, test products C and D, were positively evaluated in terms of important aspects of sensations experienced during use of a lotion, i.e., moistness, clarity, and smoothness. Some subjects commented that the color of test product D, of which 0.5% was accounted for by C18-adsorbed fraction, was too dark as a lotion.

Test 2: Skin-whitening effects

Nine subjects were divided into 3 groups each consisting of 3 subjects. These groups were designated as follows: a group to which a control product and test product B were to be applied; a group to which a control product and test product C were to be applied; and a group to which a control product and test product D were to be applied.
One of the arms of each subject was coated with a control product, and the other arm was coated with a given test product. Adequate amounts of the products were applied once a day, and this procedure was continued for 2 weeks.
The subjects were requested to reply to an inquiry survey 2 weeks after the initiation of the test. In the survey, the score of the control product was designated as point 0. The subjects were requested to grade each test product from -3 to 3 by points, with higher scores for products of more effective whitening effects, and the sum was determined to evaluate the products. The results are shown in Table 6.

Table 6

| Results of inquiry survey concerning skin-whitening effects | | | |
|---|---|---|---|
| | Group to which test product B was applied | Group to which test product C was applied | Group to which test product D was applied |
| Subject 1 | 0 | 2 | 2 |
| Subject 2 | 1 | 2 | 0 |
| Subject 3 | 0 | 0 | 1 |
| Total | 1 | 4 | 3 |

As shown in Table 6, lotions containing C18-adsorbed fractions, and in particular, test products C and D, were found to have skin-whitening effects.
There was no difference in skin-whitening effects between test product C, of which 0.1% was accounted for by C18-adsorbed fractions, and test product D, of which 0.5% was accounted for by C18-adsorbed fractions. In view of the opinion that the color of the test product D was too dark as a lotion in Test 1, it is adequate for the content of the C18-adsorbed fractions in a lotion be about 0.1 %.

[Example 11]

Test of anthocyanidin compound for inhibiting tyrosinase activity

Example 5 demonstrated that glycosides of anthocyanidin compounds contained in acerola (cyanidin-3-rhamnoside and pelargonidin-3-rhamnoside) had activity of inhibiting tyrosinase activity. In this Example, effects of various compounds shown in Table 7 for inhibiting tyrosinase activity were inspected.

Table 7

| Samples | Sources |
|---|---|
| Cyanidin-3-rhamnoside | Purified from acerola |
| Pelargonidin-3-rhamnoside | Purified from acerola |
| Quercetin-3-rhamnoside | Purified from acerola |
| Isoquercitrin | Purified from acerola |
| Astilbin | Purified from acerola |
| Hyperoside | Purified from acerola |
| Cyanidin chloride | Manufactured by Alexis Corporation |
| Cyanidin-3-glucoside | Purified from purple maze powder (manufactured by San-Ei Gen F. F. I., Inc.) |
| Pelargonidin chloride | Manufactured by Extrasynthese |
| Delphinidin chloride | Manufactured by Extrasynthese |
| Peonidin chloride | Manufactured by Extrasynthese |
| Malvidin chloride | Manufactured by Extrasynthese |
| Quercetin | Manufactured by Wako Pure Chemical Industries, Ltd. |
| Kojic acid | Manufactured by Wako Pure Chemical Industries, Ltd. |

Among the compounds shown in Table 7, cyanidin-3-rhamnoside, pelargonidin-3-rhamnoside, quercetin-3-rhamnoside, isoquercitrin, astilbin, and hyperoside are acerola-derived polyphenols. Cyanidin, pelargonidin, delphinidin, peonidin, and malvidin are anthocyanidin compounds. Isoquercitrin is another name for quercetin-3-glucoside, astilbin is another name for dehydroquercitrin, and hyperoside is another name for quercetin-3-galactoside.

The sample compounds were dissolved in DMSO and diluted with DMSO at adequate concentrations.

1/15M phosphate buffer (70 $\mu$l), 70 $\mu$l of a solution containing mushroom tyrosinase adjusted at 1 U/$\mu$l with a buffer, and 2 $\mu$l each of sample solutions dissolved in DMSO at various concentrations were introduced into separate wells of a 96-well plate, and these substances were thoroughly mixed, followed by incubation at 25°C for 10 minutes.

A solution of 10 mM L-Dopa (70 $\mu$l) was added and mixed in immediately thereafter. Changes in the absorbance at 475 nm were assayed every 20 seconds for 2 minutes. The reaction was carried out at 25°C.

Change (slope) in the absorbance per unit time was designated as the initial rate, and the inhibition of tyrosinase activity (%) was determined by the following equation.

$$\text{Inhibition (\%)} = \{1 - (\text{initial rate}_{sample}/\text{initial rate}_{control})\} \times 100$$

The concentration at which 50% of tyrosinase activity was inhibited was determined and designated as $IC_{50}$. The $IC_{50}$ figures for the samples are shown in Table 8.

Table 8

| | $IC_{50}$ ($\mu$M) |
|---|---|
| Cyanidin-3-rhamnoside | 33 |
| Pelargonidin-3-rhamnoside | 5.7 |
| Quercetin-3-rhamnoside | n.d.[a] |
| Isoquercitrin | > $2.0 \times 10^3$ |
| Astilbin | > $2.0 \times 10^3$ |
| Hyperoside | > $2.0 \times 10^3$ |
| Cyanidin chloride | 11 |
| Cyanidin-3-glucoside | 84 |
| Pelargonidin chloride | 20 |
| Delphinidin chloride | 16.4 |
| Peonidin chloride | 0.6 |
| Malvidin chloride | 0.4 |
| Quercetin | 3.0 |

(continued)

| | $IC_{50}$ (μM) |
|---|---|
| Kojic acid | 38 |

n.d.[a] Not detected

Anthocyanidin compounds, cyanidin and pelargonidin, were found to have effects of inhibiting tyrosinase activity as glycoside-forms and as free-forms (chloride salts). Other anthocyanidin compounds, delphinidin, peonidin, and malvidin, were also found to have effects of inhibiting tyrosinase activity as free-forms (chloride salts). Effects of peonidin and malvidin were found to be particularly potent.

Among the cyanidin glycosides, acerola-derived cyanidin-3-rhamnoside ($IC_{50}$: 33 μM) was found to be a more potent inhibitor of tyrosinase activity, compared with cyanidin-3-glucoside ($IC_{50}$: 84 μM) which is known to be contained in black beans and the like.

Although quercetin was found to have potent effects of inhibiting tyrosinase activity ($IC_{50}$: 3.0 μM) as an educt, no effect of inhibiting tyrosinase activity was observed for quercetin as a glycoside. Quercetin differed completely from anthocyanidin compounds in this respect.

All publications, patents, and patent applications cited herein are incorporated herein by reference in their entirety.

## Claims

1. An inhibitor of melanin formation comprising, as an active ingredient, an acerola-derived polyphenol compound.

2. An inhibitor of melanin formation comprising, as an active ingredient, an acerola polyphenol fraction obtained from acerola juice or acerola extract.

3. An inhibitor of melanin formation comprising, as an active ingredient, an anthocyanidin compound, a glycoside thereof (excluding cyanidin-3-glucoside), or a physiologically acceptable salt thereof.

4. The inhibitor of melanin formation according to claim 3, wherein the anthocyanidin compound is at least one member selected from the group consisting of cyanidin, pelargonidin, delphinidin, malvidin, and peonidin.

5. The inhibitor of melanin formation according to claim 3 or 4, wherein the glycoside comprises an anthocyanidin compound and, bound thereto via a glycoside bond, a monosaccharide selected from the group consisting of rhamnose, glucose, galactose, mannose, xylose, ribose, arabinose, and glucuronic acid or an oligosaccharide comprising a plurality of the monosaccharides, which may be the same or different.

6. The inhibitor of melanin formation according to any one of claims 1 to 5, which further comprises, as an active ingredient, ascorbic acid or an ascorbic acid derivative.

7. A skin-whitening agent comprising, as an active ingredient, the inhibitor of melanin formation according to any one of claims 1 to 6.

8. A cosmetic composition comprising the skin-whitening agent according to claim 7.

9. A food or beverage composition comprising the skin-whitening agent according to claim 7.

10. A pharmaceutical composition comprising the skin-whitening agent according to claim 7.

11. A food or beverage composition comprising an acerola-derived polyphenol compound in an amount of 0.1 g to 30 g per 100 g thereof.

12. A food or beverage composition comprising an acerola-derived polyphenol compound and ascorbic acid or an ascorbic acid derivative, wherein the proportion of the weight of the acerola-derived polyphenol compound to the weight of the ascorbic acid or ascorbic acid derivative is 5:1 to 1:10.

# Fig. 1

# Fig. 2

# Fig. 3

# Fig. 4

## INTERNATIONAL SEARCH REPORT

| International application No. |
|---|
| PCT/JP2005/015009 |

**A. CLASSIFICATION OF SUBJECT MATTER**
*A61K8/97* (2006.01), *A61K8/49* (2006.01), *A61Q19/00* (2006.01), *A23L1/30* (2006.01), *A61K31/375* (2006.01), *A61K36/00* (2006.01), *A61P17/00* (2006.01), *A61K51/00* (2006.01)
According to International Patent Classification (IPC) or to both national classification and IPC

**B. FIELDS SEARCHED**

Minimum documentation searched (classification system followed by classification symbols)
*A61K8/97* (2006.01), *A61K8/49* (2006.01), *A61Q19/00* (2006.01), *A23L1/30* (2006.01), *A61K31/375* (2006.01), *A61K36/00* (2006.01), *A61P17/00* (2006.01), *A61K51/00* (2006.01)

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
JAPICDOC(JOIS), JCATALOG(JOIS), JCHEM(JOIS), JMEDPlus(JOIS), JST7580(JOIS), JSTPlus(JOIS)

**C. DOCUMENTS CONSIDERED TO BE RELEVANT**

| Category* | Citation of document, with indication, where appropriate, of the relevant passages | Relevant to claim No. |
|---|---|---|
| X | JP 2000-212032 A (Kose Corp.), 02 August, 2000 (02.08.00), Claims; Par. No. [0006]; tables 1, 2 (Family: none) | 1-8 |
| X | JP 2004-238345 A (Suzuko UECHI), 26 August, 2004 (26.08.04), Claims; Par. Nos. [0029], [0064] (Family: none) | 1-8 |
| X Y | JP 2-200610 A (Nichirei Corp.), 08 August, 1990 (08.08.90), Claims (Family: none) | 8 1-12 |

☒ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 November, 2005 (29.11.05) | 13 December, 2005 (13.12.05) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

| International application No. |
| --- |
| PCT/JP2005/015009 |

C (Continuation).　DOCUMENTS CONSIDERED TO BE RELEVANT

| Category* | Citation of document, with indication, where appropriate, of the relevant passages | Relevant to claim No. |
| --- | --- | --- |
| Y | TSUDA T et al., Inhibition of Tyrosinase activity by antocyanin pigments isolated from Phaseolus vulgaris L., Food Sci Technol Int Tokyo, Vol.3, No.1, 1997, pages 82 to 83, particularly, tables 1, 2 | 1-12 |
| Y | HANAMURA T et al., "Acerola (Maloighia glabra L) Kajitsu ni Fukumareru Polyphenol Seibun no Kozo Kaiseki", Annual Meeting of JSBBA Koen Yoshishu, Vol.2004, page 82, 05 March, 2004 (05.03.04), page 82, 2A21p26 | 1-12 |
| X<br>Y | JP 3-099090 A  (INVERNI DELLA BEFFA SPA), 24 April, 1991 (24.04.91), Claims; particularly, Claims 2, 12, 13; examples 1 to 4<br>& EP 412300 A2　　　　& US 5200186 A<br>& CA 2022962 A　　　　& IT 1231725 B | 8-10<br>1-12 |
| X<br>Y | JP 2004-099578 A  (Oriza Yuka Kabushiki Kaisha), 02 April, 2004 (02.04.04), Claims; Par. Nos. [0002], [0004] (Family: none) | 3-10<br>1-12 |
| X | JP 2003-508415 A  (UNIV MICHIGAN STATE), 04 March, 2003 (04.03.03), Claims; particularly, Claims 2, 12, 13; examples 1 to 4<br>& WO 200115553 A1　　　& US 6818234 B1<br>& KR 2002042652 A　　　& CN 1384713 A | 9 |
| X | JP 2001-046030 A  (Tama Biochemical Co., Ltd.), 20 February, 2001 (20.02.01), Claims; Par. Nos. [0002], [0003], [0021] (Family: none) | 9-10 |
| X | JP 2003-108979 A  (Rohto Pharmaceutical Co., Ltd.), 21 January, 2003 (21.01.03), Claims; examples 2 to 4 (Family: none) | 9-10 |
| X | JP 2001-328941 A  (Yoshiaki TANIGUCHI), 27 November, 2001 (27.11.01), Claims; Par. No. [0028] (Family: none) | 9-10 |
| X | WO 2003/033005 A1  (RUDOLF WILD GMBH & CO KG), 24 April, 2003 (24.04.03), Claims; particularly, Claims 6, 9 to 10; table 1, No.9<br>& DE 10150824 A1　　　& EP 1435983 A1<br>& US 2005/002961 A1　　& JP 2005-506353 A | 9 |

Form PCT/ISA/210 (continuation of second sheet) (April 2005)

Form PCT/ISA/210 (continuation of second sheet) (April 2005)

**INTERNATIONAL SEARCH REPORT**

| International application No. |
| --- |
| PCT/JP2005/015009 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | JP 62-153211 A (Shiseido Co., Ltd.),<br>08 July, 1987 (08.07.87),<br>Claims<br>(Family: none) | 8 |
| X | JP 62-048611 A (Shiseido Co., Ltd.),<br>03 March, 1987 (03.03.87),<br>Claims<br>(Family: none) | 8,10 |
| P,X | JP 2005-154432 A (Nichirei Corp.),<br>16 June, 2005 (16.06.05),<br>Claims<br>(Family: none) | 9-10 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2814094 B
- JP 10316533 A
- JP 2003375913 A
- JP 2003375323 A
- JP 2003314207 A
- JP 2004238702 A

**Non-patent literature cited in the description**

- **F. A. BADRIA ; M. A. EL GAYYAR.** *Boll. Chim. Farmac. - Anno,* 2001, vol. 140 (4), 267-271